(19) Europäisches Patentamt
European Patent Office
Office européen des brevets

(11) **EP 3 922 621 B1**

(12) **EUROPÄISCHE PATENTSCHRIFT**

(45) Veröffentlichungstag und Bekanntmachung des Hinweises auf die Patenterteilung:
**30.08.2023   Patentblatt 2023/35**

(21) Anmeldenummer: **20179366.8**

(22) Anmeldetag: **10.06.2020**

(51) Internationale Patentklassifikation (IPC):
$C07C\ 1/04$ (2006.01)        $C07C\ 1/24$ (2006.01)
$C07C\ 11/04$ (2006.01)      $C07C\ 11/06$ (2006.01)
$C07C\ 29/141$ (2006.01)    $C07C\ 31/10$ (2006.01)
$C07C\ 45/50$ (2006.01)      $C07C\ 47/02$ (2006.01)
$C07C\ 4/02$ (2006.01)

(52) Gemeinsame Patentklassifikation (CPC):
(C-Sets verfügbar)
C07C 11/04; C07C 1/24; C07C 4/02; C07C 11/06;
C07C 29/141; C07C 31/10; C07C 45/50;
C07C 47/02; Y02P 20/141; Y02P 20/582    (Forts.)

(54) **PROZESS ZUR HERSTELLUNG VON PROPYLEN**

PROCESS FOR MANUFACTURING PROPYLENE

PROCÉDÉ DE FABRICATION DE PROPYLÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**15.12.2021   Patentblatt 2021/50**

(73) Patentinhaber: **Linde GmbH**
**82049 Pullach (DE)**

(72) Erfinder:
• **Haidegger, Ernst**
  **85521 Riemerling (DE)**
• **Meiswinkel, Andreas**
  **83253 Rimsting (DE)**
• **Zander, Hans-Jörg**
  **81479 München (DE)**

(74) Vertreter: **Reuß, Stephanie**
**Linde GmbH**
**Intellectual Property EMEA**
**Dr.-Carl-von-Linde-Straße 6-14**
**82049 Pullach (DE)**

(56) Entgegenhaltungen:
BR-A- PI0 604 284      US-B1- 9 856 198
US-B2- 10 519 087

(52) Gemeinsame Patentklassifikation (CPC): (Forts.)

C-Sets
**C07C 1/24, C07C 11/06;**
**C07C 4/02, C07C 11/04;**
**C07C 29/141, C07C 31/10;**
**C07C 45/50, C07C 47/02**

**Beschreibung**

Die vorliegende Erfindung betrifft einen Prozess zur Herstellung von Propylen den Oberbegriffen der unabhängigen Patentansprüche.

Hintergrund der Erfindung

Die Herstellung von Propylen (Propen) ist in der Fachliteratur, beispielsweise in dem Artikel "Propylene" in Ullmann's Encyclopedia of Industrial Chemistry, Ausgabe 2012, beschrieben. Propylen wird herkömmlicherweise durch Dampfspalten (Steam Cracking) von Kohlenwasserstoffeinsätzen und Umwandlungsverfahren im Zuge von Raffinerieprozessen hergestellt. In letzteren Verfahren wird Propylen nicht notwendigerweise in der gewünschten Menge und nur als eine von mehreren Komponenten in einem Gemisch mit weiteren Verbindungen gebildet. Andere Verfahren zur Herstellung von Propylen sind ebenfalls bekannt, aber nicht in allen Fällen, beispielsweise hinsichtlich Effizienz und Ausbeute, zufriedenstellend.

Für die Zukunft wird ein steigender Bedarf an Propylen prognostiziert ("Propylen-Gap"), der die Bereitstellung entsprechender selektiver Verfahren erfordert. Gleichzeitig gilt es Kohlendioxidemissionen zu reduzieren oder ganz zu verhindern. Als potentielle Ausgangsverbindung stehen andererseits große Mengen an Methan bereit, die derzeit nur sehr begrenzt einer stofflichen Verwertung zugeführt und überwiegend verbrannt werden. In entsprechenden Erdgasfraktionen sind zudem oft nennenswerte Mengen an Ethan vorhanden.

Die US10519087B2 offenbart ein Verfahren zur Herstellung von Propanal in einer Reaktion, die die oxidative Kopplung von Methan (OCM) und Sauerstoff als Reaktantenstrom in einer Gasphasenreaktion umfasst, vorzugsweise in Gegenwart von Wasser oder Dampf, um Ethylen, Ethan, Kohlendioxid, Wasser und Synthesegas in einem ersten Reaktor als Ethylenstrom zu bilden, und dann Propanal in einem zweiten Reaktor zu bilden, indem der Ethylenstrom mit dem Synthesegas aus dem ersten Reaktor in der Gasphase in den zweiten Reaktor eingespeist und in Gegenwart eines Katalysators für eine Wassergasshiftreaktion hydroformyliert wird. Bei dem Verfahren wird das Verhältnis von Wasserstoff zu Kohlenmonoxid im Synthesegas aufrechterhalten, indem entweder Dampf in den ersten Reaktor oder in den zweiten Reaktor mit eingespeist wird, um zusätzlichen Wasserstoff im Synthesegas zu erzeugen, oder indem im zweiten Reaktor Kohlenmonoxid in der Wassergssshiftreaktion gebildet wird, indem das Kohlendioxid aus dem Ethylenstrom in den zweiten Reaktor eingespeist wird. BRPI 0604284 offenbart ein Verfahren zur Herstellung von Propylen über Ethylen, Propanal und Propanol. Das Ethylen wird durch Dehydratisierung von Bio-Ethanol gewonnen. US 9856198 offenbart einen ähnlichen Sachverhalt, geht aber von Ethylen aus.

Die vorliegende Erfindung stellt sich die Aufgabe, ein insbesondere in Anbetracht dieser Aspekte verbessertes Verfahren zur Herstellung von Propylen bereitzustellen.

Offenbarung der Erfindung

Vor diesem Hintergrund schlägt die vorliegende Erfindung einen Prozess zur Herstellung von Propylen mit den jeweiligen Merkmalen der unabhängigen Patentansprüche vor. Bevorzugte Ausgestaltungen der vorliegenden Erfindung sind Gegenstand der abhängigen Patentansprüche sowie der nachfolgenden Beschreibung.

Grundsätzlich existiert neben den zuvor erwähnten Dampfspaltverfahren eine Vielzahl von unterschiedlichen Verfahren zur Umwandlung von Kohlenwasserstoffen und verwandten Verbindungen ineinander, von denen nachfolgend einige exemplarisch genannt werden sollen.

Beispielsweise ist die Umwandlung von Paraffinen zu kettenlängengleichen Olefinen durch die oxidative Dehydrierung (ODH, im Falle von Ethan auch als ODHE bezeichnet) bekannt. Typischerweise wird bei der ODH auch eine kettenlängengleiche Carbonsäure, bei der ODHE also Essigsäure, als Koppelprodukt gebildet. Ethylen kann aber auch durch oxidative Kopplung von Methan (OCM) hergestellt werden. Über eine Kettenverlängerung, beispielsweise durch die unten beschriebene Hydroformylierung, kann man auch ausgehend von dem in der oxidativen Dehydrierung von Ethan gebildeten Ethylen zu Propylen gelangen.

Die Herstellung von Propylen aus Propan durch Dehydrierung (PDH) ist ebenfalls bekannt und stellt ein kommerziell verfügbares und etabliertes Verfahren dar. Ebendies gilt für die Herstellung von Propylen aus Ethylen durch die Olefinmetathese. Dieses Verfahren erfordert 2-Buten als zusätzliches Edukt.

Schließlich existieren sogenannte Methane-to-Olefine- bzw. Methane-to-Propylene-Verfahren (MTO, MTP), in denen aus Methan zunächst Synthesegas hergestellt wird und das Synthesegas anschließend zu Olefinen wie Ethylen und Propylen umgesetzt wird. Entsprechende Verfahren können auf Grundlage von Methan, jedoch auch auf Grundlage von anderen Kohlenwasserstoffen oder kohlenstoffhaltigen Ausgangsmaterialien wie Kohle oder Biomasse betrieben werden.

Als Einzeltechnologien sind die Dampfreformierung und die Trockenreformierung sowie Abwandlungen hiervon inklusive einer nachgeschalteten Wassergasshift zur Einstellung des Verhältnisses von Wasserstoff zu Kohlenmonoxid ebenfalls bekannt.

Zur Synthesegasherstellung ist ferner die Partialoxidation (POX), insbesondere bei höherem Kohlenmonoxidanteil, bekannt. Synthesegase können auch durch eine Vergasung von diversen Einsätzen (Kohle, Öl, Abfälle, insbesondere Kunststoffabfälle etc.) erzeugt werden.

Die bereits zuvor im Zusammenhang mit der oxidativen Dehydrierung von Ethan erwähnte Hydroformylierung stellt eine weitere Technologie dar, die insbesondere zur Herstellung von so genannten Oxoverbindungen genutzt wird. Typischerweise wird in der Hydroformylierung Propylen umgesetzt, es können jedoch auch höhere Kohlenwasserstoffe, insbesondere Kohlenwasserstoffe mit sechs bis elf Kohlenstoffatomen, oder Ethylen eingesetzt werden. Diverse Verfahren zur Hydrierung von Aldehyden und Dehydratisierung von Alkoholen (insbesondere von Ethanol und Propanol) sind ebenfalls bekannt.

Beispielsweise kann zur Herstellung von Propylen, wie erwähnt, eine Hydroformylierung im Anschluss an eine oxidative Dehydrierung von Ethan eingesetzt werden. Im Produktstrom der oxidativen Dehydrierung sind dabei unter anderem gewisse Anteile an Kohlenmonoxid sowie insbesondere noch nicht umgesetztes Ethan enthalten. Insbesondere der hohe Anteil von Ethan im Bereich von 25 bis 70 Molprozent, insbesondere von 30 bis 50 Molprozent, führt zu einer signifikanten Verdünnung und beeinflusst die Partialdrücke in der Hydroformylierung erheblich. Ferner wird in der oxidativen Dehydrierung von Ethan Kohlendioxid gebildet, das abgetrennt und ggf. gesondert behandelt werden muss. Hierfür kann beispielsweise eine Trockenreformierung eingesetzt werden. Eine nennenswerte Integration der Aufreinigungs- und Zerlegungsschritte ist dabei bisher nicht bekannt und die bisher in Betracht gezogenen Verfahrensschritte sind auf den Spezialfall einer oxidativen Dehydrierung von Ethan und der Verwendung eines noch nicht aufgereinigten Produktstromes sowie die Verwertung des Nebenproduktes Kohlendioxid in der Trockenreformierung beschränkt.

Auch eine Propylenherstellung ausgehend von einem Komponentengemisch, das neben Ethylen auch erhebliche Anteile an Methan, Kohlenmonoxid und Kohlendioxid sowie ggf. auch Wasserstoff und Ethan enthält, und das beispielsweise durch eine oxidative Kopplung von Methan bereitgestellt werden kann, kann grundsätzlich erfolgen. Auch hier kann die weitere Verarbeitung eine Hydroformylierung umfassen. Auch hier kommt es jedoch zu einer signifikanten Verdünnung der Reaktanden, die die Partialdrücke in der Hydroformylierung erheblich beeinflussen. Ein typischer Ethylengehalt liegt in einem Produktgemisch aus der oxidativen Kopplung von Methan grundsätzlich eher am unteren Ende eines Bereichs von 5 bis 60 Molprozent. Die Hydroformylierung erfolgt bevorzugt bei hohem Druck und insbesondere Methan wirkt hier als Inertmedium, welches die Partialdrücke der Reaktionspartner Ethylen, Kohlenmonoxid und Wasserstoff negativ beeinflusst. Zudem wirkt sich die vergleichsweise hohe Verdünnung überdies auf die Größe der Apparate negativ aus und verteuert damit die Investitions- und Betriebskosten. Entsprechende Nachteile können z.B. durch die Bildung von Kohlendioxid auch grundsätzlich dann auftreten, wenn eine Shiftreaktion zur Konvertierung von Kohlenmonoxid in Wasserstoff vorgenommen wird.

Entsprechend beschreibt auch die US10,519,087 B2 ein Verfahren, bei dem ein Einsatzstrom aus einer oxidativen Kopplung von Methan verwendet wird und somit, wie erläutert, neben Ethylen erhebliche Mengen anderer Komponenten, insbesondere leichte und/oder inerte Komponenten enthält, die dann durch die Hydroformylierung geführt werden müssen. Auf die soeben erläuterten Nachteile wird verwiesen.

Die vorgenannten Verfahren verwenden allesamt einen Rohgasstrom, der neben den Komponenten Kohlenmonoxid, Wasserstoff und Ethylen auch weitere Komponenten enthält. Ein entsprechender Rohgasstrom enthält nicht notwendigerweise die richtige Zusammensetzung für die nachfolgenden Verfahrensschritte und kann typischerweise auch inerte Komponenten wie Methan, Ethan sowie ggf. Kohlendioxid, aber auch störende Verbingungen wie z.B. Acetylen, Oxygenate etc. enthalten. Daher ist typischerweise in solchen Verfahren ein nicht unerheblicher Reinigungs- und Trennaufwand erforderlich.

Merkmale und Vorteile der Erfindung

Die vorliegende Erfindung schafft ein Verfahren, das es ermöglicht zur Deckung des Eingangs erwähnten, zunehmenden Bedarfs an Propylen, insbesondere auch im Verhältnis zu Ethylen, beizutragen. Sie ermöglicht insbesondere eine Einbindung von Methan oder anderen niedrigpreisigen Kohlenstoffquellen in die petrochemische Wertschöpfungskette in einer gegenüber dem Stand der Technik und den erläuterten grundsätzlich ebenfalls möglichen Verfahren vorteilhafter Weise. Hierdurch ist insbesondere eine Verwertung von bislang nicht genutzten Begleitgasen aus der Erdöl-und Erdgasförderung oder auch aus der Vergasung von Abfällen, insbesondere Kunststoffabfällen, möglich. Dies kann zu einer deutlichen Reduzierung des Kohlendioxid-Footprints (petro-)chemischer Anlagen führen.

Bestehende Technologien wie Dampfspalten, Methane-to-Olefine- bzw. Methane-to-Propylene-Verfahren produzieren Ethylen und Propylen immer nur in einem bestimmten Verhältnis, welches sich in Abhängigkeit vom Einsatz durch Anpassung der Prozessparameter (z.B. Temperatur, Verweilzeit etc.) nur bedingt einstellen lässt. Hier ermöglicht die vorliegende Erfindung deutliche Verbesserungen und ermöglicht eine gezielte Einstellung bzw. Fokussierung auf ein Zielolefin wie Propylen.

Die Olefinmetathese, die zwar eine flexible Umwandlung von Ethylen in Propylen ermöglicht, geht heutzutage von den Olefinen Ethylen und 2-Buten aus, die zu Propylen umgesetzt werden. Es handelt sich prinzipiell um eine Gleichgewichtsreaktion, und ursprünglich wurde diese Technologie für die umgekehrte Umsetzung, also von Propylen zu Ethylen und Buten entwickelt. Das notwendige 2-Buten kann z.B. aus der selektiven Dimerisierung von Ethylen oder als Fraktion aus der Produktion von linearen α-Olefinen, ebenfalls basierend auf der Oligomerisierung von Ethylen, gewonnen werden. Ebenfalls kann 1-Buten eingesetzt werden, welches isomerisiert werden kann. Ebenso ist auch die Verwendung von höheren internen linearen Olefinen bekannt, wobei sich durch entsprechende Reaktionskaskaden letztlich schlussendlich immer Propylen als Zielprodukt ergibt.

Auch wenn die Technologie der Olefinmetathese als ausgreift und etabliert angesehen werden kann, bleibt als ein wesentlicher Nachteil, dass bereits ausschließlich hochwertige und hochpreisige Edukte verwendet werden. Die Olefinmetathese dient daher zumeist insbesondere dazu, eine flexible Ausbilanzierung von Ethylen- und Propylenproduktion und -bedarf an einem spezifischen Standort oder Verbund zu ermöglichen. Die vorliegende Erfindung ist im Gegensatz zur Olefinmetathese vorteilhaft, weil sie von einfachen, kostengünstigen Ausgangsprodukten wie üblichen Feeds für ein Dampfspaltverfahren und Methan ausgehen kann.

Außer sogenannten MTO oder MTP-Verfahren (Wertschöpfungskette: Methan, Synthesegas, Methanol, Propylen) existiert bislang kein direkter Weg von Methan bzw. Erdgas hin zu Propylen. Beispielhaft sei insbesondere das Lurgi-MTP-Verfahren angeführt, welches jedoch ca. 20-30% benzinartige Verbindungen als niedrig bewertetes Beiprodukt erzeugt. Die vorliegende Erfindung vermeidet dies.

Im Gegensatz zu herkömmlichen Verfahren werden im Rahmen der vorliegenden Erfindung deutlich geringere Mengen an Kohlendioxid erzeugt, und zwar einerseits durch die reduzierte Befeuerung, da dies im Gegensatz zu endothermen Prozessen wie (reinem) Dampfspalten und Propandehydrierung nicht oder nur in geringerem Umfang erforderlich ist, und andererseits, da kein Kohlendioxid als Nebenprodukt gebildet wird, wie beispielsweise in der oxidativen Dehydrierung.

In einer bevorzugten Ausführungsform vereinfacht die vorliegende Erfindung die Abtrennung von Nebenprodukten wie Wasserstoff, Methan, Kohlenmonoxid und Kohlendioxid sowie weiterer Kohlenwasserstoffen beim Dampfspalten und von Kohlenmonoxid und Kohlendioxid bei der oxidativen Dehydrierung signifikant bzw. macht eine separate Abtrennung überflüssig.

Weitere Vorteile der vorliegenden Erfindung ergeben sich damit in bevorzugten Ausgestaltungen insbesondere hinsichtlich der Aufreinigung und Fraktionierung. Bekannte Wege zur Olefinherstellung erfordern oft eine aufwändige, insbesondere kryogene Trennung, z.B. Ethan/Ethylen für das Dampfspalten und die oxidative Dehydrierung, von Kohlenwasserstoffen mit zwei und drei bzw. mit drei und mehr Kohlenstoffatomen für das Dampfspalten sowie von Propan/Propylen für das Dampfspalten und die Propandehydrierung. Die vorliegende Erfindung ermöglicht in einer bevorzugten Ausführungsform insbesondere eine Integration von Prozessschritten, insbesondere von besonders aufwändigen Schritten wie kryogenen Trennungen (z.B. in C1/C2-Trennungen, C2/C3-Trenungen, C3/C4-Trennungen und sogenannten C2- und C3-Splittern). Insbesondere die praktisch quantitative Abtrennung einer Fraktion, die Methan und ggf. leichtere Verbindungen enthält, bzw. einer Fraktion, die Ethan und ggf. leichtere Verbindungen enthält, von höheren Kohlenwasserstoffen ist in den etablierten Prozessen zumeist erforderlich und erfordert entsprechende (kryogene) Apparate. In einer bevorzugten Ausführungsform erleichtert die vorliegende Erfindung diese Schritte.

Die vorliegende Erfindung schlägt zur Erzielung der genannten Vorteile einen Prozess zur Herstellung von Propylen vor, bei dem unter Verwendung eines Dampfspaltverfahrens und einer Fraktionierung ein erster Stoffstrom bereitgestellt wird, der reich an Ethylen ist, und bei dem unter Verwendung eines Synthesegaserzeugungsverfahrens ein zweiter Stoffstrom, der Kohlenmonoxid und Wasserstoff enthält, bereitgestellt wird.

Die Bereitstellung des ersten und zweiten Stoffstroms kann beliebige, insbesondere typischerweise dem Dampfspaltverfahren bzw. dem Synthesegaserzeugungsverfahren zugeordnete Trenn- und Aufbereitungsschritte umfassen, beispielsweise Hydrierungs-und Fraktionierungsschritte bekannter Art bzw. eine Abtrennung bestimmter Komponenten. Grundsätzlich können das Dampfspalt- und das Synthesegaserzeugungsverfahren in beliebiger, aus dem Stand der Technik bekannter Weise und mit beliebigen Einsätzen und Reaktionsbedingungen durchgeführt werden. Der erste, an Ethylen reiche Stoffstrom kann das Ethylen insbesondere in einem Gehalt von mehr als beispielsweise 90 oder 95 oder 99 Molprozent enthalten oder in einer definierten Reinheit. Beispielsweise kann es sich um sogenanntes "Polymer Grade"-Ethylen handeln, wie es üblicherweise durch Dampfspalten und anschließende Fraktionierung gewonnen werden kann. Der zweite Stoffstrom kann Kohlenmonoxid und Wasserstoff insbesondere ebenfalls in einem entsprechenden Gehalt aufweisen.

Beispielsweise kann das Dampfspaltverfahren zur Herstellung von Ethylen optimiert sein und mit entsprechenden Reaktionsbedingungen und Einsätzen betrieben werden. Besonders vorteilhafte Beispiele werden unten erläutert. Insbesondere können das Dampfspaltverfahren und das Synthesegasherstellungsverfahren auch aufeinander abgestimmt betrieben werden, insbesondere zur Anpassung der jeweiligen Produkte in einem geeigneten stöchiometrischen Verhältnis zueinander.

Beispielsweise kann das Synthesegasherstellungsverfahren eine Vergasung beliebiger Einsätze, beispielsweise von Kunststoffabfällen oder dergleichen, umfassen. Auch eine Herstellung von Synthesegas aus gasförmigen Einsätzen, insbesondere von Methan, beispielsweise aber auch unter Einsatz einer Dampfreformierung, ist möglich. Es kann auch beispielsweise eine sogenannte Trocken- oder Kohlendioxidreformierung erfolgen. Zur Synthesegaserzeugung können grundsätzlich alle Verfahren verwendet werden, sofern sie ein vorteilhaftes Wasserstoff-Kohlenmonoxid-Verhältnis (idealerweise 1:1 nur für die Hydroformylierung und 2:1 für den Gesamtprozess mit Hydroformylierung und Hydrierung, sofern kein Wasserstoff aus anderer Quelle zur Verfügung steht) liefern. Entsprechend wird hier der Begriff Synthesegaserzeugung verwendet, welcher insbesondere die Dampfreformierung, Trocken- bzw. Kohlendioxidreformierung, partielle Oxidation und Vergasungsverfahren umfassen soll und daher als übergreifende Definition verwendet wird. Ebenfalls kann die erfindungsgemäße Synthesegaserzeugung optional weitere Schritte wie eine Wassergasshift zur Justierung des Wasserstoff-Kohlenmonoxid-Verhältnisses oder eine Reinigung (z.B. Aminwäsche, insbesondere Rectisolwäsche) beinhalten.

Im Rahmen der vorliegenden Erfindung wird zumindest ein Teil des Ethylens aus dem ersten Stoffstrom mit zumindest einem Teil des Kohlenmonoxids und des Wasserstoffs aus dem zweiten Stoffstrom unter Verwendung einer Hydroformylierung unter Erhalt eines dritten Stoffstroms zu Propanal umgesetzt. Zumindest ein Teil des Propanals in dem dritten Stoffstrom wird im Rahmen der vorliegenden Erfindung zu Propylen umgesetzt. Das Ethylen wird mittels des Dampfspaltverfahrens in einem ersten Komponentengemisch bereitgestellt, das Propylen wird in einem zweiten Komponentengemisch bereitgestellt.

Hierbei wird der erste Stoffstrom dabei aus einer Fraktionierung erhalten, welcher das erste Komponentengemisch oder ein Teil hiervon unterworfen wird, und die hier als "erste" Fraktionierung bezeichnet wird. In der ersten Fraktionierung werden vorteilhafterweise weitere Stoffströme gebildet. Die weiteren Stoffströme können insbesondere auch einen Reinpropylenstrom umfassen, der in dem Dampfspaltverfahren gebildetes Propylen aufweist.

Zumindest ein Teil des zweiten Komponentengemischs wird in der Erfindung, jeweils unter Erhalt eines Reinpropylenstromes, entweder einem oder mehreren Fraktionierungsschritten der erwähnten ersten Fraktionierung, oder einer separaten Fraktionierung, hier als "zweite" Fraktionierung bezeichnet, unterworfen.

Entsprechend können erfindungsgemäß also auch ein, zwei oder mehrere Fraktionierungen mit beliebigen Trennschritten vorgesehen sein, die in geeigneter Weise jeweils das Propylen aus dem ersten, aus dem zweiten und/oder aus einem kombinierten Komponentengemisch als ein oder mehrere Reinpropylenströme gewinnen. Auch eine gemeinsame Fraktionierung, die zwei oder mehr getrennte Trennschritte zur Bereitstellung mehrerer Reinpropylenströme aufweisen kann, kann in entsprechender Weise verwendet werden. Bei Verwendung einer gemeinsamen Fraktionierung können dabei beliebige Fraktionierungsschritte mehrfach, insbesondere parallel, vorhanden sein, und dabei gemeinsam oder getrennt voneinander mit Stoffströmen aus den jeweiligen Verfahren gespeist werden.

Die Fraktionierung kann dabei neben Fraktionierungsschritten, die konkret zur Bereitstellung des ersten Stoffstroms bzw. des oder der Reinpropylenströme verwendet werden, weitere Fraktionierungsschritte beliebiger Art umfassen. Bei den Fraktionierungsschritten kann es sich beispielsweise um eine Demethanisierung, eine Deethanisierung, eine Depropanisierung, eine Debutanisierung, eine Trennung zwischen Kohlenwasserstoffen mit zwei (und ggf. weniger) Kohlenstoffatomen und drei (und ggf. mehr) Kohlenstoffatomen voneinander, eine Trennung von unterschiedlichen Kohlenwasserstoffen mit zwei Kohlenstoffatomen (insbesondere von Ethan und Ethylen, sog. C2-Splitter) voneinander und/oder eine Trennung von unterschiedlichen Kohlenwasserstoffen mit drei Kohlenstoffstoffatomen (insbesondere von Propan und Propylen, sog. C3-Splitter) voneinander umfassen, die in beliebiger Reihenfolge durchgeführt werden können. Auch eine Hydrierung bestimmter Komponenten kann stromauf oder stromab beliebiger Fraktionierungsschritte vorgesehen sein. Der erste Stoffstrom wird insbesondere in einer entsprechenden Trennung von unterschiedlichen Kohlenwasserstoffen mit zwei Kohlenstoffstoffatomen bereitgestellt, ein oder mehrere Reinpropylenströme können dagegen in einer entsprechenden Trennung von unterschiedlichen Kohlenwasserstoffen mit drei Kohlenstoffstoffatomen bereitgestellt werden. Diese Trennungen können, wie erwähnt, ein- oder mehrfach vorhanden sein und in beliebigen Trennsequenzen gruppiert werden.

Besonders bevorzugt ist eine Ausführungsform, bei der zumindest ein Teil des ersten und zumindest ein Teil des zweiten Komponentengemischs unter Erhalt des ersten Stoffstroms und eines gemeinsamen Reinpropylenstroms einer gemeinsamen, d.h. der erwähnten ersten Fraktionierung unterworfen werden. In diesem Fall ist nicht notwendigerweise eine weitere Fraktionierung vorhanden.

Die vorliegende Erfindung ermöglicht durch die erfindungsgemäß vorgeschlagenen Maßnahmen eine bedarfsgerechte Überführung von Ethylen in Propylen unter Verwendung von bislang stofflich nahezu ungenutztem Methan oder anderen geeigneten Kohlenstoffquellen. Diese Überführung kann für den gesamten Ethylen-Produktstrom eines Dampfspaltverfahrens oder aber auch nur für einen Teilstrom des Ethylenproduktes erfolgen. In Summe und bei idealisierter Betrachtung wird dabei aus einem Molekül Ethylen und einem weiteren Kohlenstoffatom ein Molekül Propylen gewonnen. Das weitere Kohlenstoffatom kann dabei je nach Verfahren zur Synthesegaserzeugung aus Methan (insbesondere bei der Dampfreformierung und der partiellen Oxidation) bzw. aus Methan und Kohlendioxid (insbesondere bei der Trocken- bzw. Kohlendioxidreformierung) oder einer anderen Quelle (insbesondere bei einem Vergasungsverfahren) stammen. Prinzipiell ist auch eine (anteilige) Verwertung von höheren Kohlenwasserstoffen, beispielsweise in einer Dampfreformierung und einer partiellen Oxidation, möglich.

In dem erfindungsgemäß vorgeschlagenen Prozess umfasst die Umsetzung zumindest eines Teils des Propanals zu Propylen insbesondere eine Hydrierung zu Propanol und eine Dehydratisierung zu Propylen.

Die grundsätzliche Prozessabfolge der Hydroformylierung von Ethylen, Hydrierung und Dehydratisierung ist zwar grundsätzlich bekannt, jedoch ist die Hydroformylierung von Ethylen in der Praxis bislang kaum relevant. Im Rahmen der vorliegenden Erfindung werden nun die Technologien und Prozessschritte Dampfspalten inklusive typischerweise enthaltener bzw. notwendiger Verdichtungs-, Reinigungs- und Trennschritte etc. sowie Synthesegaserzeugung (Dampfreformierung oder alternative Verfahren), Hydroformylierung, Hydrierung und Dehydratisierung vorteilhaft miteinander kombiniert und integriert, so dass sich durch den Einsatz der Erfindung ein besonders vorteilhaftes und effizientes integriertes Verfahren ergibt.

Insbesondere kann der dritte Stoffstrom nicht umgesetztes Ethylen, Wasserstoff und Kohlenmonoxid als leichte Komponenten enthalten, wobei die leichten Komponenten in einer Abtrennung zumindest zu einem Teil in einen vierten Stoffstrom überführt werden. Die leichten Komponenten können insbesondere in einer an sich bekannten, in gängigen Fraktionierungseinrichtungen vorhandenen C2/C3-Trennung abgetrennt werden. Schwerere Komponenten können insbesondere in einer an sich bekannten C3/C4-Trennung abgetrennt werden.

Die Abtrennung zumindest eines Teils der leichten Komponenten in der erläuterten Weise kann in unterschiedlichen Ausgestaltungen der vorliegenden Erfindung entweder vor der Hydrierung oder zwischen der Hydrierung und der Dehydratisierung erfolgen, wozu die entsprechenden Prozessschritte in der Reihenfolge vertauscht werden können.

Der vierte Stoffstrom oder ein Teil davon kann insbesondere als Recylestrom stromauf der Hydroformylierung in den Prozess zurückgeführt werden. Die Rückführung kann dabei insbesondere in den Eintritt der Hydroformylierung erfolgen. Je nach Bedarf kann zur Druckanpassung bzw. zur Kompensation von Druckverlusten eine geeignete Nachverdichtung bzw. Druckanpassung erfolgen. Sofern eine (nahezu bzw. im Wesentlichen) vollständige Umsetzung von Kohlenmonoxid und Wasserstoff in der Hydroformylierung erfolgt, was durch eine geeignete Anpassung der Stöchiometrieverhältnisse und Verfahrensbedingungen erfolgen kann, kann auf eine entsprechende Bildung eines Recyclestroms auch verzichtet werden.

Um die Akkumulation von Spuren und Inerten zu vermeiden, kann bei Bedarf ein Teil des vierten Stoffstroms als ein Purgestrom zurück zur Synthesegaserzeugung geführt werden. Unter (möglicherweise hypothetischen) idealen Bedingungen, also insbesondere dann, wenn keine Anteile von Methan, Ethan und/oder Kohlendioxid in einem Produktstrom der Hydroformylierung vorliegen und keine Bildung inerter Nebenprodukte erfolgt, könnte ein entsprechender Purgestrom auch theoretisch ganz entfallen. Bei einem geringen Eintrag und/oder Bildung der genannten Nebenprodukte kann der Purgestrom entsprechend klein dimensioniert werden.

Wie erwähnt, können im Rahmen der vorliegenden Erfindung grundsätzlich bekannte Maßnahmen zur Abtrennung zumindest eines Teils der leichten Komponenten eingesetzt werden. Diese können insbesondere eine adsorptive Trennung, eine absorptive Trennung, eine Membrantrennung, eine destillative Trennung und/oder eine Phasentrennung umfassen. Unter einer "Phasentrennung" soll dabei eine reine Trennung von flüssiger Phase und Gasphase nach Abkühlung in einem keine typischen Trenneinrichtungen einer Rektifikationskolonne aufweisenden Trennbehälter verstanden werden.

Insbesondere kann beispielsweise eine Extraktivdestillation zum Einsatz kommen, bei der ein Teil des gebildeten Propanols als ein Extraktionsmittel verwendet werden kann. Auf diese Weise können insbesondere kryogene Bedingungen vermieden werden. Aufgrund des minimierten apparativen Aufwandes kann auch ein reiner Abscheider, bei dem nach Abkühlung auf moderate (nicht-kryogene) Temperaturen das Produkt der Hydroformylierung (Propanal) als Flüssigphase anfällt, besonders vorteilhaft sein.

Allgemein sollen hier unter "nicht-kryogenen" Bedingungen solche Bedingungen verstanden werden, die ein Temperaturniveau von nicht weniger als -20 °C, insbesondere nicht weniger als 0 °C, insbesondere nicht weniger als Kühlwassertemperatur von 5 bis 25 °C, umfassen. Bei einer reinen Phasentrennung in der Flüssigphase gelöste leichtere Moleküle sind dabei nicht störend, da diese durch die eine weitere Integration wie unten ausgeführt abgetrennt und einer weiteren stofflichen Verwertung zugeführt werden können.

In dem erfindungsgemäßen Prozess wird vorteilhafterweise in die Hydrierung ein Wasserstoffstrom zugeführt, der Wasserstoff enthält, welcher unter Verwendung einer separaten Wasserstoffquelle bereitgestellt und/oder unter Verwendung des Synthesegaserzeugungsverfahrens und/oder unter Verwendung des Dampfspaltverfahrens gewonnen und unter Verwendung einer Wasserstoffabtrennung abgetrennt wird. Beispielsweise kann entsprechender Wasserstoff aus dem Synthesegas der Synthesegaserzeugung gewonnen werden. In einer Alternative kann es sich um Wasserstoff aus einer entsprechenden Leichtgasfraktion handeln, die in dem Dampfspaltverfahren anfällt.

Ein an den leichten Komponenten abgereicherter, an Propanal reicher Stoffstrom, beispielsweise ein an Propanal reicher flüssiger Stoffstrom aus einer entsprechenden Phasentrennung, kann im Rahmen der vorliegenden Erfindung in die Hydrierung geführt werden. Bei der anschließenden Dehydratisierung des in der Hydrierung gebildeten Propanols bildet sich Wasser. Dieses fällt in einem Produktstrom an, der das Propylen und die Nebenkomponenten enthält. Dieser kann im Rahmen der vorliegenden Erfindung unter Erhalt eines das Propylen und die Nebenkomponenten enthaltenden und an Wasser abgereicherten Teilstroms einer Wasserabtrennung unterworfen werden.

Die Wasserfraktion kann bei Bedarf z.B. zur Erzeugung von Prozessdampf genutzt werden. Ein nach der Wasserabtrennung verbleibender Rest enthält Kohlenwasserstoffe (insbesondere und zum überwiegenden Teil das Propylen) und ggf. Spuren von Kohlenmonoxid, Wasserstoff und beispielsweise Propanal, Propanol und weitere Oxygenate sowie höhere Kohlenwasserstoffe.

Der verbleibende Rest, also ein das Propylen und die Nebenkomponenten enthaltender und an Wasser abgereicherter Teilstrom oder ein in einer optionalen und bei Bedarf vorgesehenen Spurenentfernung aus diesem Teilstrom gebildeter Folgestrom kann unter Erhalt einer Reinpropylenfraktion einer oder mehreren bereits zuvor erwähnten Fraktionierungen bzw. Fraktionierungsschritten einer oder mehrerer Fraktionierungen unterworfen werden. Zu weiteren Erläuterungen sei auf die obigen Ausführungen zur "ersten" und "zweiten" Fraktionierung verwiesen.

In einer besonders bevorzugten Ausführungsform wird einer einzigen ("ersten") Fraktionierung ganz oder teilweise das in dem Dampfspaltverfahren erzeugte Produktgemisch zugeführt, wie erläutert. Dabei wird neben der Reinpropylenfraktion auch der erste Stoffstrom ganz oder teilweise erhalten. Anders ausgedrückt wird der das Propylen und die Nebenkomponenten enthaltende und an Wasser abgereicherte Teilstrom oder der in der optionalen und bei Bedarf vorgesehenen Spurenentfernung aus diesem Teilstrom gebildete Folgestrom in Form des zweiten Komponentengemischs einer gemeinsamen ("ersten") Fraktionierung mit dem im Dampfspaltverfahrens erhaltenen ersten Komponentengemisches unterworfen.

Eine Kombination beider Kompoenentengemische - erforderlichenfalls auch nur jeweiliger Teilströme - erfolgt entsprechend dem Druckniveau an geeigneter Stelle. Auf diese Weise kann insbesondere der Trenn- und Aufreinigungsaufwand für die Herstellung von Reinpropylen verringert werden. Die Zuführung des ersten und zweiten Komponentengemischs oder entsprechender Teile hiervon kann an unterschiedlichen Stellen der Fraktionierung erfolgen, je nach der Zusammensetzung der Komponentengemische. Das Ethylen, also der erste Stoffstrom, kann insbesondere einem sogenannten C2-Splitter - der evtl. auch bereits im Rahmen des Dampfspaltverfahrens vorhanden ist - das Reinpropylen dagegen einem sogenannten C3-Splitter - der evtl. ebenfalls bereits auch im Rahmen des Dampfspaltverfahrens vorhanden ist - entnommen werden, wie ebenfalls bereits erwähnt. Der dabei erhaltene Reinpropylenstrom kann daher bei Verwendung einer einzigen Fraktionierung anteilig Propylen aus dem Dampfspaltverfahren und anteilig Propylen aus der Sequenz Hydroformylierung, Hydrierung und Dehydratisierung umfassen. Die Erfindung kann dabei insbesondere auch die Parallelisierung von einzelnen oder mehreren Baugruppen, wie z.B. Trennungen, C2- und C3-Splitter umfassen, wenn eine Paralleliseirung aufgrund z.B. der Prozessführung, Baubarkeit und/oder Skalierbarkeit erforderlich ist.

In dieser besonders bevorzugten Ausgestaltung der Erfindung können die erforderlichen Aufreinigungen und Trennungen ganz oder teilweise in einer gemeinsamen Fraktionierung durchlaufen werden, wodurch unerwünschte Komponenten abgetrennt werden können. Eine Trennung von Propylen und evtl. als Nebenprodukt einer Überhydrierung entstandenem Propan kann beispielsweise in einem ohnehin vorhandenen C3-Splitter erfolgen. Schwerere Komponenten können zuvor, falls vorhanden, von Propan und Propylen abgetrennt werden. Das abgetrennte Propan kann wiederum dem Dampfspaltverfahren ganz oder teilweise als Einsatzstoff zur Verfügung gestellt werden. Auch höhere Kohlenwasserstoffe (mit beispielsweise vier und mehr Kohlenstoffatomen) können auf diese Weise wahlweise ganz oder teilweise jeweils als separate Stoffströme aus dem Gesamtprozess ausgeführt und verwertet, oder aber wiederum dem Dampfspaltverfahren ganz oder teilweise als Einsatzstoff zur Verfügung gestellt werden. Hierdurch können auch evtl. anfallende Spuren- oder Nebenprodukte aus der Prozesskette Hydroformylierung, Hydrierung und Dehydratisierung mit vier oder mehr Kohlenstoffatomen einer stofflichen Verwertung zugeführt werden. Dies gilt sinngemäß für weitere Verbindungen mit entsprechendem Siedeverhalten, z.B. insbesondere Alkohole.

Im Zuge des Gesamtprozesses werden also im Idealfall alle anfallenden Nebenströme über eine entsprechende Rückführung stofflich verwertet, was einen besonderen Vorteil der vorliegenden Erfindung darstellt.

Allgemein kann in der vorliegenden Erfindung insbesondere Methan sinnvoll stofflich genutzt werden, welches oft als "Begleitgas", z.B. bei der Erdölförderung, anfällt und bisher nicht stofflich verwertet wird. Damit ist dieses unmittelbar mit der Gewinnung von entsprechenden Dampfspalteinsätzen wie Naphtha, sogenanntem Atmospheric Gasoil (AGO) oder Vaccum Gasoil (VGO) verbunden. Auch bei der Nutzung von leichten Fraktionen wie Ethan, Propan und Butan bzw. sogenanntem Liquefied Petroleum Gas (LPG) als Dampfspalteinsatz müssen zuvor die zumeist enthaltenen erheblichen Methananteile abgetrennt werden. Sofern keine Einspeisung z.B. in Erdgaspipelines, möglich ist, wird das Methan herkömmlicherweise zumeist verbrannt. Das resultierende Kohlendioxid ist zwar weniger klimaschädlich als Methan, trägt jedoch immer noch in erheblichem Maße zur Erderwärmung bei. Die vorliegende Erfindung vermeidet dies durch die beschriebene Verwendung des Synthesegaserzeugungsverfahrens, in dem das Methan genutzt werden kann, in dem vorgeschlagenen Gesamtprozess. Alternativ ermöglicht die Verwendung von Synthesegas aus Vergasungsverfahren die stoffliche Nutzung von Abfällen, insbesondere Kunststoffabfällen.

Andere Verfahren zur (selektiven) Propylenherstellung setzen, wie bereits oben dargelegt, oft bereits hochwertige Produkte um. So werden, wie bereits erwähnt, in der Olefinmetathese bereits hochwertige Produkte (Olefine, insbesondere Ethylen und 2-Buten) zu Propylen umgesetzt, während beim vorgeschlagenen Verfahren das Upgrade von Ethylen zu Propylen aus der Verwendung von Methan erfolgt (bei einem idealen Verhältnis der Kohlenstoffbilanz mit zwei Kohlenstoffatomen aus Ethylen und einem Kohlenstoffatom aus Methan). Sogenannte On-Purpose-Verfahren wie die Propandehydrierung ergeben zwar im wesentlichen Propylen als Produkt, jedoch können hier ebenfalls anfallende leichtere oder schwerere Fraktionen nicht bzw. nicht selektiv zur Propylengewinnung genutzt werden.

Das hier beschriebene Verfahren erlaubt hingegen die Nutzung aller dieser Fraktionen in der Dampfspaltung mit den entsprechenden nachgeschalteten Verfahrensschritten und dem Synthesegaserzeugungsverfahren sowie eine bedarfsgerechte und flexible Anpassung des Verhältnisses von Ethylen zu Propylen. Auf diese Weise kann also Ethylen bedarfsgerecht und kostengünstig in Propylen überführt werden. Dabei ergeben sich besondere Vorteile durch die Integration von Prozessschritten und der gemeinsamen Nutzung ohnehin vorhandener und/oder zu errichtender Prozessschritte wie z.B. Verdichtungen, Reinigungen, Trocknung und Trennungen (insbesondere umfassend einen C3-Splitter, der einen erheblichen apparativen Aufwand darstellt), wie in Ausgestaltungen der Erfindung der Fall. Diese Schritte sind bislang üblicherweise dem Dampfspaltverfahren als eigenständige Trennsequenz nachgeschaltet.

Der Wirkungsgrad des Prozesses kann als Olefineffizienzfaktor $O_{Ef}$ bzw. Monomerwirkungsgrad MWG in % angegeben werden. Er ergibt sich zunächst als Quotient aus der Masse an über die Reaktionskaskade Hydroformylierung, Hydrierung und Dehydratisierung gewonnenem Reinpropylen $m_{Reinpropylen}$ und der Masse an in der Hydroformylierung eingesetztem Ethylen $m_{Ethylen}$ wie folgt:

$$O_{Ef} = MWG = 100\% * m_{Reinpropylen} / m_{Ethylen}$$

Unter idealisierten Bedingungen (also jeweils 100% Umsatz mit 100% Selektivität) beträgt dieser Wert maximal 150%. Der Olefineffizienzfaktor $O_{Ef}$ bzw. Monomerwirkungsgrad MWG läßt sich auch aus den entsprechenden Einzelschritten des Verfahrens ableiten. Dabei ist jeder Einzelschritt (i = 1 bis x) - also insbesondere die Hydroformylierung, eine Trennung von leichten Komponenten, die Hydrierung, die Dehydratisierung und eine Fraktionierung - charakterisiert durch ein Paar von Umsatz $X_i$ und Selektivität $S_i$, welches unter technischen Bedingungen optimalerweise erreicht werden kann. Daraus ergibt sich die jeweilige Ausbeute des Einzelschrittes:

$$Y_i = X_i * S_i.$$

Dabei kann für eine Trennung und eine Fraktionierung der Umsatz jeweils auf 100% gesetzt werden, da hier zwar keine Reaktion von einem Edukt zu einem andersartigen Produkt erfolgt erfolgt, aber die Eintrittsmasse gleich der Austrittsmasse für jede Komponente sein muss. Edukt und Produkt sind bei dieser Betrachtung für eine Trennung bzw. Fraktionierung also identische Komponenten. Die Selektivität ist hier sodann ein Mass für den Wirkungsgrad der Trennung bzw. Fraktionierung. Bei einer Selektivität von 100% gelangt die entsprechende Komponente vollständig in die Zielfraktion, während bei Werten kleiner als 100% ein Verlust in eine jeweils andere Fraktion der Trennung bzw. Fraktionierung erfolgt. Werte kleiner als 100% quantifizieren also den entsprechenden Verlust der jeweiligen Komponente, der nicht in die Zielfraktion gelangt.

Es gilt zunächst für die Gesamtausbeute $Y_{Total}$ des Prozesses bezogen auf Ethylen und den daraus weiter gebildeten Reaktionsprodukten:

$$Y_{Total} = \prod_{i=1}^{x} Y_i \prod_{i=1}^{x} Y_i$$

Bei Rückführung von nicht umgesetzten Edukten durch Recycle in den entsprechenden Reaktionsschritt (insbesondere z.B. nicht umgesetztes Ethylen in der Hydroformylierung) ergibt sich ggf. ein höherer Umsatz für den entsprechenden Einzelschritt; es ist also dann ein entsprechend angepaßter höherer Wert $X_i$ bzw. $Y_i$ für diesen Schritt anzusetzen.

Der Olefineffizienzfaktor bzw. Monomerwirkungsgrad ist abhängig von der Effizienz jedes einzelnen Verfahrensschrittes und läßt sich durch Multiplikation von $Y_{Total}$ mit dem Molmassenverhältnisses von Propylen zu Ethylen berechnen:

$$O_{Ef} = MWG = 100\% * Y_{Total} * (M_{C3H6} / M_{C2H4}) = 100\% \prod_{i=1}^{x} Y_i * (M_{C3H6} / M_{C2H4})$$

Aus den entsprechenden Einzelschritten und deren charakteristischen Werten für $X_i$, $S_i$ und $Y_i$ läßt sich ableiten, dass der Olefineffizienzfaktor bzw. Monomerwirkungsgrad in einer bevorzugten Ausführung bestehend aus Hydroformylierung, Trennung von leichten Komponenten, Hydrierung, Dehydratisierung und Fraktionierung mindestens 100%, insbesondere mindestens 115%, 125% oder 130%, und bis zu 150%, beträgt.

Ein vergleichbarer Olefineffizienzfaktor $O_{Ef}$(Metathese) läßt sich auch für die Olefinmetathese berechnen, hier sind jedoch sowohl Ethylen als auch Buten - welches häufig aus der Dimerisierung von Ethylen gewonnen wird - als hochwertige olefinische Edukte einzubeziehen:

$$O_{Ef}(\text{Metathese}) = 100\% * m_{Reinpropylen} / (m_{Ethylen} + m_{Buten})$$

Definitionsgemäß ist der Olefineffizienzfaktor $O_{Ef}$(Metathese) für die Olefinmetathese daher also immer <100% und erreicht nur im Idealfall 100%. Der Olefineffizienzfaktor bzw. Monomerwirkungsgrad $O_{Ef}$ > 100% veranschaulicht den besonderen Vorteil sowie den praktischen Nutzen der vorliegenden Erfindung, da erfindungsgemäß besonders bevorzugt Werte für den Olefineffizienzfaktor $O_{Ef}$ höher als 100% erzielt werden.

In einer besonders vorteilhaften Ausgestaltung des erfindungsgemäßen Verfahrens kann in das Synthesegaserzeugungsverfahren, das dazu geeignet ausgestaltet ist, Kohlendioxid eingespeist werden. Dies kann insbesondere bei einer Dampf- oder Kohlendioxid- bzw. Trockenreformierung erfolgen. Hierdurch wird ein erhöhter Anteil an Kohlenmonoxid im Austrittsstrom des Reformers erreicht. Eine solche Variante erscheint insbesondere bei Dampfspaltverfahren mit leichtem Einsatz (Ethan, Propan, Butan, LPG) vorteilhaft, da hier ohnehin im Spaltprozess eine signifikante Menge an Wasserstoff anfällt und als separater Wasserstoffstrom für die Hydroformylierung und/oder Hydrierung bereitgestellt werden kann. Kohlendioxid kann jedoch auch aus einer externen Quelle stammen (rein oder im Gemisch mit Methan) bzw. auch aus Kohlendioxidwäschen, also typischerweise Aminwäschen. Hier kann insbesondere eine Entfernung aus den Abgas-/Rauchgasen, wie sie bei der Beheizung mit Brennern anfallen, vorteilhaft sein, um so den Kohlendioxid-Footprint eines integrierten Gesamtprozesses zu minimieren. Ebenfalls ist so die Nutzung von Kohlendioxid als Nebenprodukt im Prozessgas des Dampfspaltverfahrens möglich.

In einer weiteren vorteilhaften Ausgestaltung umfasst der Prozess, dass in dem Dampfspaltverfahren und/oder Synthesegaserzeugungsverfahren eine elektrische Beheizung durchgeführt wird, und dass in das Synthesegaserzeugungsverfahren Methan eingespeist wird, das in dem Dampfspaltverfahren gebildet wird. Mit anderen Worten kann Methan, das ganz oder teilweise aus einer entsprechenden Fraktion aus dem Dampfspaltverfahren stammt, unmittelbar in die Synthesegaserzeugung gefahren werden. Auf diese Weise kann ein entsprechender Stoffstrom, der ansonsten zur Befeuerung der Öfen verwendet wird, stofflich verwertet werden.

Zusätzlich ist der (teilweise) elektrische Betrieb von Antrieben von z.B. Pumpen, Kompressoren und Turbinen möglich, um einen zusätzlichen Dampfbedarf des Gesamtprozesses zu minimieren oder komplett zu vermeiden. Auf diese Weise ist eine weitere Minimierung des Kohlendioxidausstoßes möglich, d.h. es muss kein kohlenstoffhaltiger Rohstoff zur Dampferzeugung verbrannt werden, sondern dieser kohlenstoffhaltige Rohstoff kann teilweise oder vollständig in einen geeigneten, gemäß einer vorteilhaften Ausgestaltung der Erfindung vorgesehenen Schritt zur Synthesegaserzeugung geführt werden.

Im Falle eines Vergasungsverfahrens zur Synthesegaserzeugung bietet die vollständige oder teilweise Nutzung von Kunststoffabfällen ("Plastikrecycling") eine nutzbringende Wiederverwertung solcher Abfälle im Sinne einer nachhaltigen Kreislaufwirtschaft.

In einer Ausgestaltung der Erfindung kann der Prozess auch umfassen, dass der erste Stoffstrom unter Verwendung einer dem Dampfspaltverfahren zugeordneten Tailend-Hydrierung gebildet wird und Wasserstoff enthält. Besonders vorteilhaft erscheint eine Verfahrensvariante in der die C2-Hydrierung des Steamcrackers als so genannte Tailend-Hydrierung erfolgt. Eine Tailend-Hydrierung bezeichnet eine Hydrierung einer gebildeten Fraktion und nicht eines stromauf vorliegenden Gesamtgemischs. Die Hydrierung erfolgt zur Umsetzung von Acetylen(en). Im Rahmen der vorliegenden Erfindung kann dabei ein Überschuss nach der Tailend-Hydrierung toleriert werden, ohne dass eine zusätzliche Feinreinigung des ersten Stoffstroms erforderlich ist. Dieser Vorteil kommt dann voll zum Tragen, wenn das gesamte Ethylen aus dem Cracker in dem erfindungsgemäßen Verfahren verwertet wird, da dann kein Teilstrom als eigenständiges Endprodukt aufgereinigt werden muss. Das erfindungsgemäße Verfahren benötigt in der Hydroformylierung ohnehin Wasserstoff und Kohlenmonoxid.

Der erwähnte, das Propylen und die Nebenkomponenten enthaltende und an Wasser abgereicherte Teilstrom oder ein in einer optionalen und bei Bedarf vorgesehenen Spurenentfernung aus diesem Teilstrom gebildeter Folgestrom, der bei niedrigem Druck anfällt, kann beispielsweise vor eine entsprechende Verdichterstufe in einer sogenannten Rohgashydrierung des Dampfspaltverfahrens auf geeignetem Druckniveau geführt werden. Es kann sich insbesondere um die zweite Stufe handeln. Anstatt eine separate Wasserabreicherung vorzunehmen, kann diese ebenfalls in einer dem Dampfspaltverfahren zugeordneten Fraktionierung erfolgen, die typischerweise stromauf der Rohgasverdichtung erfolgt. Hierdurch ergibt sich ein entsprechender Druckverlust nach der Dehydratisierung, die allerdings bereits typischerweise auf relativ niedrigem Druckniveau erfolgt.

An beliebiger Stelle kann Abwärme aus der Synthesegaserzeugung für das Dampfspaltverfahren, insbesondere zur Vorwärmung und/oder Dampferzeugung bzw. Bereitstellung von Niederdruckdampf, verwendet werden.

Prinzipiell kann das eingesetzte Ethylen ganz oder anteilig auch aus jeder beliebigen Quelle stammen, die Ethylen in entsprechender Reinheit bereitstellt (z.B. Ethanoldehydratisierung, oxidative Dehydierung, MTO/MTP-Verfahren). Aufgrund der möglichen Integration mit dem vorangegangenen Verfahren ergibt sich besondere Relevanz jedoch für solche Verfahren, bei denen in der vorangeschalteten Olefinerzeugung ebenfalls eine Auftrennung der C3-Fraktion enthalten ist (z.B. Dampfspaltung, MTO/MTP-Verfahren).

Eine Anlage zur Herstellung von Propylen, mit einer Dampfspalteinrichtung und einer Fraktionierung, die zur Durchführung eines Dampfspaltverfahrens und zur Bereitstellung eines Ethylen enthaltenden ersten Stoffstroms eingerichtet ist, mit einer Synthesegaserzeugungseinrichtung, die zur Durchführung eines Synthesegaserzeugungsverfahrens und zur Bereitstellung eines Kohlenmonoxid und Wasserstoff enthaltenden zweiten Stoffstroms eingerichtet ist, und mit einer Hydroformylierungseinrichtung, die dafür eingerichtet ist, zumindest einen Teil des Ethylens aus dem ersten Stoffstrom mit zumindest einem Teil des Kohlenmonoxids und des Wasserstoffs aus dem zweiten Stoffstrom unter Verwendung einer Hydroformylierung unter Erhalt eines dritten Stoffstroms zu Propanal umzusetzen, kann zur Durchführung des vorgeschlagenen Verfahrens verwendet werden, wobei die Anlage ferner dafür eingerichtet ist, das Ethylen mittels des Dampfspaltverfahrens in einem ersten Komponentengemisch bereitzustellen, zumindest einen Teil des Propanals in dem dritten Stoffstrom zu Propylen umzusetzen und hierbei ein das Propylen enthaltendes zweites Komponentengemisch bereitzustellen. Wie bereits in Bezug auf das erfindungsgemäß vorgeschlagene Verfahren erläutert, kann vorgesehen sein, zumindest einen Teil des ersten und zumindest einen Teil des zweiten Komponentengemischs unter Erhalt des ersten Stoffstroms und eines oder mehrerer Reinpropylenströme jeweils einer oder mehreren Fraktionierungen bzw. Fraktionierungsschritten zu unterwerfen.

Eine entsprechende Anlage ist insbesondere zur Durchführung eines Verfahrens eingerichtet, wie es zuvor in unterschiedlichen Ausgestaltungen erläutert wurde. Auf die entsprechenden Erläuterungen, die auch die erfindungsgemäß vorgeschlagene Anlage betreffen, kann daher an dieser Stelle verwiesen werden.

Die Erfindung wird nachfolgend unter Bezugnahme auf spezifische Beispiele und die beigefügten Zeichnungen näher erläutert, welche bevorzugte Ausgestaltungen der vorliegenden Erfindung veranschaulichen.

Beispiele

Wie bereits ausgeführt, kommen für die Synthesegasgaserzeugung verschiedene Varianten (Dampfreformierung, Trocken- bzw. Kohlendioxidreformierung, Partialoxidation, Vergasungsverfahren) und auch Kombinationen insbesondere von Dampfreformierung und Trockenreformierung in Betracht. In idealisierter Weise beschreiben die nachfolgenden Gleichungen I, III und IV die jeweilige Umsetzung von Methan, während die Gleichungen II, IV und VI beispielhaft die jeweilige Umsetzung von Ethan widergeben. Zusätzlich ist das resultierende $H_2/CO$-Verhältnis angegeben.

Dampfreformierung:

$$CH_4 + H_2O \rightarrow CO + 3\,H_2 \quad\quad (I) \quad\quad H_2/CO = 3{:}1$$

$$C_2H_6 + 2\,H_2O \rightarrow 2\,CO + 5\,H_2 \quad\quad (II) \quad\quad H_2/CO = 2{,}5{:}1$$

Trockenreformierung:

$$CH_4 + CO_2 \rightarrow 2\,CO + 2\,H_2 \quad\quad (III) \quad\quad H_2/CO = 1{:}1$$

$$C_2H_6 + 2\,CO_2 \rightarrow 4\,CO + 3\,H_2 \quad\quad (IV) \quad\quad H_2/CO = 0{,}75{:}1$$

Partialoxidation (POX):

$$2\,CH_4 + O_2 \rightarrow 2\,CO + 4\,H_2 \quad\quad (V) \quad\quad H_2/CO = 2{:}1$$

$$C_2H_6 + O_2 \rightarrow 2\,CO + 3\,H_2 \quad\quad (VI) \quad\quad H_2/CO = 1{,}5{:}1$$

Neben diesen Reaktionen I bis VI können auch weitere Reaktionen ablaufen, wie z.B. Wassergasshift und reverse Wassergasshift entsprechend den Gleichungen VIIa und VIIb oder insbesondere im Falle der Partialoxidation eine Totaloxidation zu Kohlendioxid und Wasser. Dies führt dann zu Abweichungen vom hier skizzierten idealen Verhalten und Verschiebungen im Verhältnis von Wasserstoff zu Kohlenmonoxid.

Wassergasshift:

$$CO + H_2O \rightarrow H_2 + CO_2 \quad\quad\quad (VIIa)$$

Reverse Wassergasshift:

$$H_2 + CO_2 \rightarrow CO + H_2O \quad\quad\quad (VIIb)$$

Unter realen Bedingungen ergeben sich also entsprechende Abweichungen. Die Reformierung von Methan liefert z.B. nicht exakt ein Verhältnis von Wasserstoff zu Kohlenmonoxid von 3:1, weil dieses Verhältnis eben durch die Shiftreaktion VIIa verschoben wird. Hier ist die Kennzahl $S_N$, das sogenannte Stöchiometriemodul bzw. die sogennannte Stöchiometriezahl, hilfreich.

$$S_N := \frac{x_{H_2} - x_{CO_2}}{x_{CO} + x_{CO_2}}$$

Die Stöchiometriezahl ist bei der Shiftreaktion invariant: Würde die Shiftreaktion komplett auf die Seite des Kohlenmonoxids verschoben, wäre das Produkt ein Gas ohne Kohlendioxid und mit einem Verhältnis von Wasserstoff zu Kohlenmonoxid, das der Stöchiometriezahl entspricht. Die realen Verhältnisse werden daher durch die Stöchiometriezahl besser beschrieben.

Die Reformierung von reinem Methan ohne Kohlendioxid und ohne höhere Kohlenwasserstoffe im Reaktionseinsatz liefert beispielsweise immer genau eine Stöchiometriezahl von 3. Bei Rückführung von Kohlendioxid, beispielsweise durch einen Teil des zuvor erwähnten vierten Stoffstroms (Strom 6 in den nachfolgenden

Figuren) kann dieses Kohlendioxid dann wieder in die Synthesegaserzeugung zurückgeführt werden und insbesondere bei Reformierung und Trockenreformierung entsprechend den Gleichungen III und IV konvertiert werden.

Betrachtet man nun die Einzelreaktionen Hydroformylierung (Gleichung IIX), Hydrierung (IX) und Dehydratisierung (X), ergibt sich der entsprechende jeweilige Wasserstoff- und Kohlenmonoxidbedarf wie folgt:

Hydroformylierung:
$C_2H_4 + H_2 + CO \rightarrow C_3H_6O$ (IIX) Bedarf $H_2/CO$ = 1:1 / $S_N$ =1:1
Hydrierung:
$C_3H_6O + H_2 \rightarrow C_3H_7OH$ (IX) nur Bedarf an 1 Äquivalent $H_2$
Dehydratisierung:
$C_3H_7OH \rightarrow C_3H_6 + H_2O$ (X) kein Bedarf an $H_2$ und CO

Für eine ideale Gesamtreaktion des integrierten Prozesses (Hydroformylierung, Hydrierung und Dehydratisierung) ergibt sich folgende Bruttogleichung XI und der nachfolgend angegebene Gesamtbedarf (Ges.-Bed.):

$C_2H_4 + 2\,H_2 + CO \rightarrow C_3H_6 + H_2O$ (XI) Ges.-Bed. $H_2/CO$ = 2:1 / $S_N$ =2:1

Entsprechend kann je nach Verfügbarkeit von weiterem Wasserstoff eines der genannten Verfahren zur Synthesegaserzeugung vorteilhaft sein. Ist ausreichend sonstiger Wasserstoff verfügbar, ist die Trockenreformierung besonders vorteilhaft, da diese hohe Ausbeuten an Kohlenmonoxid und einen idealen Synthesegasstrom für die Hydroformylierung liefert. Die Partialoxidation von Methan erscheint vorteilhaft, wenn hierdurch sowohl der Kohlenmonoxidbedarf als auch der Gesamtbedarf an Wasserstoff abzudecken sind.

Eine reine Dampfreformierung von Methan liefert zunächst einen Überschuss von Wasserstoff, der aber durch eine reverse Wassergasshift gemäß Gleichung VIIb wieder in Kohlenmonoxid überführt werden kann. Vorteilhafterweise wird dabei auch Kohlendioxid umgesetzt und es entsteht Wasser als weiteres Reaktionsprodukt.

Gemäß den Gleichungen II, IV und VI verringert sich das Verhältnis von Wasserstoff zu Kohlenmonoxid jeweils bei einer Einspeisung von höheren Kohlenwasserstoffen in die Synthesegaserzeugung. Dies stellt jedoch kein Problem dar, da ohnehin bei den meisten Verfahren eher zuviel Wasserstoff produziert wird, als im erfindungsgemäßen Verfahren benötigt wird. Eine bedarfsgerechte Einstellung ist dabei immer durch Wassergasshift bzw. reverse Wassergasshift gemäß den Gleichungen VIIa und VIIb möglich.

Insgesamt erscheint aber unter realen Bedingungen ein Verfahren zur Synthesegaserzeugung als besonders geeignet, welches eine Dampfreformierung und eine Trockenreformierung kombiniert und die bedarfsgerechte Stöchiometrie bereitstellt. Gegenüber einer Partialoxidation oder Vergasung besteht hier der zusätzliche Vorteil der integrierten stofflichen Verwertung von Kohlendioxid.

Insgesamt wird aus Gleichung XI offensichtlich, dass das gebildete Propylen aus Ethylen und Kohlenmonoxid gebildet wird. Da dieses Kohlenmonoxid wie ausgeführt aus Methan oder sogar anteilig aus Kohlendioxid gebildet wird, ergibt sich ein eindeutiger Wertzuwachs in der Prozesskette ausgehend von Ethylen unter Verwendung von ansonsten kaum oder nicht stofflich genutztem Methan und/oder Kohlendioxid.

Kurze Beschreibung der Zeichnungen

Figur 1 veranschaulicht einen Prozess gemäß einer bevorzugten Ausführungsform der Erfindung in Form eines schematischen Ablaufplans.

Figur 2 veranschaulicht einen Prozess gemäß einer besonders bevorzugten Ausführungsform der Erfindung in Form eines schematischen Ablaufplans.

Figur 3 veranschaulicht einen Prozess gemäß einer weiteren besonders bevorzugten Ausführungsform der Erfindung in Form eines schematischen Ablaufplans.

Ist nachfolgend von Prozess- oder Verfahrensschritten die Rede, sollen hierunter auch die jeweils für diese Verfahrensschritte verwendeten Apparate (insbesondere z.B. Reaktoren, Kolonnen, Wascheinrichtungen usw.) verstanden werden, auch wenn hierauf nicht ausdrücklich Bezug genommen wird. Allgemein gelten die einen Prozess betreffenden Erläuterungen für eine entsprechende Anlage jeweils in gleicher Weise.

Ausführliche Beschreibung der Zeichnungen

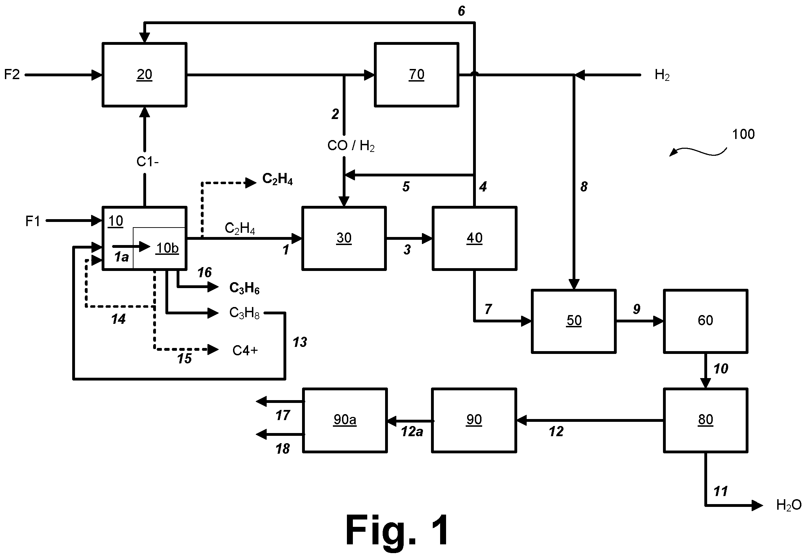

In der Figur 1 ist ein Prozess gemäß einer bevorzugten Ausgestaltung der vorliegende Erfindung in Form eines schematischen Ablaufplanes veranschaulicht und insgesamt mit 100 bezeichnet.

In dem Prozess 100 wird in einem Dampfspaltverfahren 10 unter Einsatz einer Fraktionierung 10b ein erster Stoffstrom 1 bereitgestellt, der reich an Ethylen ist. Die Fraktionierung 10b ist lediglich stark vereinfacht als gestrichelter Block und insbesondere anhand einiger dabei erhaltener Stoffströme veranschaulicht, welche in Form entsprechender Flusspfeile dargestellt sind. Der Fraktionierung 10b wird ein mit 1a bezeichnetes Produktgemisch des Dampfspaltverfahrens 10 zugeführt, das hier auch als ein "erstes Komponentengemisch" bezeichnet wird.

Beispielsweise kann in den Fraktionierungsschritten der Fraktionierung 10b eine mit C4+ bezeichnete Fraktion, die Kohlenwasserstoffe mit vier und mehr Kohlenstoffatomen enthält, in Form eines Stoffstroms 15 bereitgestellt und ganz oder teilweise in Form eines Stoffstroms 14 in das Dampfspaltverfahren 10 zurückgeführt werden. Entsprechendes gilt für eine Propanfraktion $C_3H_8$, die in Form eines Stoffstroms 13 zurückgeführt werden kann. Eine erste Reinpropylenfraktion wird als Produktfraktion 16 bereitgestellt. Dem Dampfspaltverfahren wird ein geeigneter Einsatzstrom F1 zugeführt.

Zur weiteren Erläuterungen, insbesondere bezüglich der Durchführung des Dampfspaltverfahrens 10 und der Bildung der genannten Fraktionen, kann auf gängige Fachliteratur wie den Artikel "Ethylene" in Ullmann's Encyclopedia of Industrial Chemistry, beispielsweise die Publikation vom 15 April 2009, DOI: 10.1002/14356007.a10_045.pub2, verwiesen werden.

In einem Synthesegaserzeugungsverfahren 20, das insbesondere eine Dampfreformierung und eine Trockenreformierung umfassen kann, wird unter Verwendung eines zweiten Einsatzstroms F2 ein zweiter Stoffstrom 2 bereitgestellt, der Kohlenmonoxid und Wasserstoff enthält, und der aus einem Rohproduktstrom insbesondere im Rahmen einer unten erläuterten Abtrennung von Wasserstoff bereitgestellt werden kann.

Zu möglichen Synthesegaserzeugungsverfahren 20 sei auf die obigen Erläuterungen und beispielsweise auch auf den Artikel "Gas Production" in Ullmann's Encyclopedia of Industrial Chemistry, beispielsweise die Publikation vom 15. Dezember 2006, DOI: 10.1002/14356007.a12_169.pub2, verwiesen.

Zumindest ein Teil des Ethylens aus dem ersten Stoffstrom 1 wird im hier veranschaulichten Beispiel mit zumindest einem Teil des Kohlenmonoxids und des Wasserstoffs aus dem zweiten Stoffstrom 2 unter Verwendung einer Hydroformylierung 30 unter Erhalt eines dritten Stoffstroms 3 zu Propanal umgesetzt. Der dritte Stoffstrom 3 enthält typischerweise auch nicht umgesetzte Komponenten der Stoffströme 1 und/oder 2. Zumindest ein Teil des Propanals in dem dritten Stoffstrom 3 wird unter Erhalt eines Stoffstroms 7 durch Einsatz einer Hydrierung 50 zu Propanol (Stoffstrom 9) umgesetzt und letzteres wird unter Einsatz einer Dehydratisierung 60 unter Erhalt eines Stoffstroms 10 zu dem Propylen umgesetzt.

Zumindest ein Teil des vierten Stoffstroms 4 kann als Recylestrom 5 stromauf der Hydroformylierung 30 in den Prozess 100 zurückgeführt werden. Wie erwähnt, kann dabei die Zurückführung zumindest eines Teils des vierten Stoffstroms 4 eine Nachverdichtung umfassen. Ein Teil des vierten Stoffstroms 4 kann auch zu dem Synthesegaserzeugungsverfahren 20 als Purgestrom 6 zurückgeführt werden.

Die Abtrennung 40 zumindest eines Teils der leichten Komponenten kann auf unterschiedliche Weise, im einfachsten Fall, wie erwähnt, in Form einer Abscheidung einer Flüssigfraktion, durchgeführt werden.

In die Hydrierung 50 wird ein Wasserstoffstrom 8 geführt, der unter Verwendung des Synthesegaserzeugungsverfahrens 20 gewonnen wird. Letzterer wird unter Verwendung einer Wasserstoffabtrennung 70, beispielsweise umfassend eine Druckwechseladsorption, abgetrennt. Zusätzlicher Wasserstoff $H_2$ kann bei Bedarf auch unter Verwendung einer separaten Wasserstoffquelle bereitgestellt und dem Verfahren zugeführt werden.

In der Dehydratisierung 60 wird ein Wasser, das Propylen und Nebenkomponenten enthaltender Produktstrom, der bereits zuvor erwähnte Stoffstrom 10, gebildet, der unter Erhalt eines das Propylen und die Nebenkomponenten enthaltenden und an Wasser abgereicherten Teilstroms 12 einer Wasserabtrennung 80 unterworfen wird. In der Wasserabtrennung wird ein Wasserstrom 11 gebildet, der beispielsweise zur Bereitstellung von Prozessdampf verwendet werden kann.

Der das Propylen und die Nebenkomponenten enthaltende und an Wasser abgereicherte Teilstrom 12 oder ein in einer optionalen Spurenentfernung 90 hieraus gebildeter Folgestrom 12a wird einer weiteren Fraktionierung 90a zugeführt, in der eine zweite Reinpropylenproduktfraktion 17 und ein weiterer Stoffstrom 18, der insbesondere Propan und/oder höher siedende Komponenten enthält, erhalten werden.

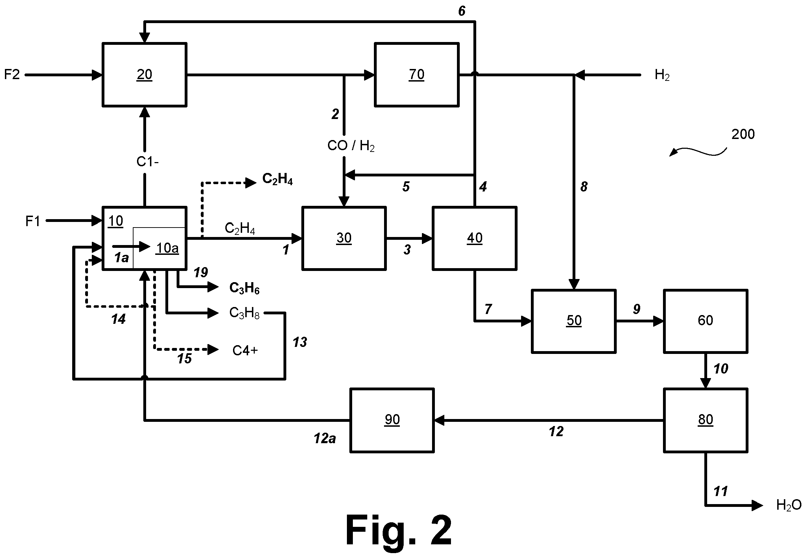

In Figur 2 ist ein Prozess gemäß einer weiteren bevorzugten Ausführungsform der Erfindung in Form eines schematischen Ablaufplans veranschaulicht und insgesamt mit 200 bezeichnet. Es gelten grundsätzlich die zuvor für Figur 1 und den Prozess 100 beschriebenen Erläuterungen. Abweichungen sind im Folgenden erläutert.

Der das Propylen und die Nebenkomponenten enthaltende und an Wasser abgereicherte Teilstrom 12 oder der in einer optionalen Spurenentfernung 90 hieraus gebildete Folgestrom 12a wird hier einer Fraktionierung 10a zugeführt, der wie in dem Prozess 100 gemäß Figur 1 auch ein in dem Dampfspaltverfahren 10 erzeugtes Produktgemisch unterworfen wird. Die Fraktionierung 10a kann wie zuvor ausgebildet oder an die spezifischen Erfordernisse der zusätzlichen Bearbeitung des Stoffstroms 12 oder des Folgestroms 12a angepasst sein. In der Fraktionierung 10a kann auf diese Weise eine gemeinsame Reinpropylenproduktfraktion 19 gebildet werden. Die Reinpropylenfraktion 19 umfasst damit Propylen aus dem Dampfspaltverfahren 10 und der Prozesskette aus Hydroformylierung 30, Hydrierung 50 und Dehydratisierung 60.

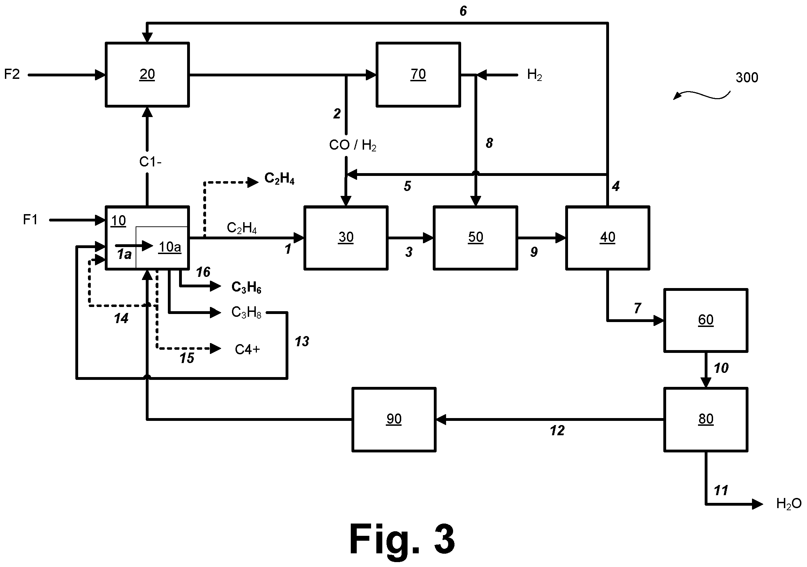

In Figur 3 ist ein Prozess gemäß einer weiteren bevorzugten Ausführungsform der Erfindung in Form eines schematischen Ablaufplans veranschaulicht und insgesamt mit 300 bezeichnet. Auch hier ist, wie in dem Verfahren 200 gemäß Figur 2, eine gemeinsame Fraktionierung 10a vorgesehen. Alternativ kann jedoch auch die in Figur 1 zu Prozess 100 dargestellte Fraktionierung 10b vorgesehen sein. Wie hier veranschaulicht, erfolgt hier Abtrennung 40, was eine besonders bevorzugte Variante darstellt, zwischen der Hydrierung 50 und der Dehydratisierung 60 erfolgen. Zu den Stoffströmen und die Bezeichnungen sei auf die obigen Ausführungen verwiesen. Wenngleich hier Stoffströme aus der Abtrennung 40 mit 7 und aus der Hydrierung mit 9 bezeichnet sind, wie zuvor, versteht sich, dass diese aufgrund der abweichenden Reihenfolge eine abweichende Zusammensetzung aufweisen. Die Bezeichnung wurde aufgrund der Art der Bereitstellung beibehalten.

## Patentansprüche

1. Prozess (100, 200, 300) zur Herstellung von Propylen, bei dem unter Verwendung eines Dampfspaltverfahrens (10) und einer oder mehrerer Fraktionierungen (10a, 10b, 90a) ein erster Stoffstrom (1) bereitgestellt wird, der reich an Ethylen ist, bei dem unter Verwendung eines Synthesegaserzeugungsverfahrens (20) ein zweiter Stoffstrom (2) bereitgestellt wird, der Kohlenmonoxid und Wasserstoff enthält, bei dem zumindest ein Teil des Ethylens aus dem ersten Stoffstrom (1) mit zumindest einem Teil des Kohlenmonoxids und des Wasserstoffs aus dem zweiten Stoffstrom (2) unter Verwendung einer Hydroformylierung (30) unter Erhalt eines dritten Stoffstroms (3) zu Propanal umgesetzt wird, und bei dem zumindest ein Teil des Propanals in dem dritten Stoffstrom (3) zu Propylen umgesetzt wird, wobei das Ethylen mittels des Dampfspaltverfahrens (10) in einem ersten Komponentengemisch bereitgestellt wird, wobei das Propylen in einem zweiten Komponentengemisch bereitgestellt wird, **dadurch gekennzeichnet, dass** zumindest ein Teil des ersten Komponentengemisch unter Erhalt des ersten Stoffstroms einer ersten Fraktionierung (10a, 10b) unterworfen wird, und dass zumindest ein Teil des zweiten Komponentengemischs unter Erhalt eines Reinpropylenstromes (19) einem oder mehreren Fraktionierungsschritte der ersten Fraktionierung (10a, 10b) oder einer zweiten Fraktionierung (90a) unterworfen wird.

2. Prozess (100, 200, 300) nach Anspruch 1, bei dem die Umsetzung zumindest eines Teils des Propanals zu dem Propylen eine Hydrierung (50) zu Propanol und eine Dehydratisierung (60) zu Propylen unter Erhalt des zweiten Produktgemischs umfasst.

3. Prozess (100, 200, 300) nach Anspruch 2, bei dem der dritte Stoffstrom (3) nicht umgesetztes Ethylen, Wasserstoff und Kohlenmonoxid als leichte Komponenten enthält, wobei die leichten Komponenten in einer Abtrennung (40) zumindest zu einem Teil in einen vierten Stoffstrom (4) überführt werden.

4. Prozess (100, 200, 300) nach Anspruch 3, bei dem die Abtrennung (40) zumindest eines Teils der leichten Komponenten vor der Hydrierung (50) oder zwischen der Hydrierung (50) und der Dehydratisierung (60) erfolgt.

5. Prozess (100, 200, 300) nach Anspruch 3 oder 4, bei dem zumindest ein Teil des vierten Stoffstroms (4) als Recylestrom (5) stromauf der Hydroformylierung (30) in den Prozess (100, 200, 300) zurückgeführt wird.

6. Prozess (100, 200, 300) nach Anspruch 4, bei dem die Zurückführung zumindest eines Teils des vierten Stoffstroms (4) eine Nachverdichtung umfasst.

7. Prozess (100, 200, 300) nach Anspruch 6, bei dem ein Teil des vierten Stoffstroms (4) zu dem Synthesegaserzeugungsverfahren (20) zurückgeführt wird.

8. Prozess (100, 200, 300) nach einem der Ansprüche 5 oder 6, bei dem die Abtrennung (40) zumindest eines Teils der leichten Komponenten eine adsorptive Trennung, eine adsorptiven Trennung, eine Membrantrennung, eine destillative Trennung und/oder eine Phasentrennung umfasst.

9. Prozess (100, 200, 300) nach einem der Ansprüche 2 bis 8, bei dem in die Hydrierung ein Wasserstoffstrom (8) zugeführt wird, der Wasserstoff enthält, der unter Verwendung einer separaten Wasserstoffquelle bereitgestellt und/oder unter Verwendung des Synthesegaserzeugungsverfahrens (20) und/oder unter Verwendung des Dampfspaltverfahrens (10) gewonnen und unter Verwendung einer Wasserstoffabtrennung (70) abgetrennt wird.

10. Prozess (100, 200, 300) nach einem der Ansprüche 2 bis 9, bei dem in der Dehydratisierung (60) ein Wasser, Propylen und Nebenkomponenten enthaltender Produktstrom (10) gebildet wird, der unter Erhalt des Propylen und ferner die Nebenkomponenten enthaltenden und an Wasser abgereicherten Teilstroms (12) einer Wasserabtrennung (80) unterworfen wird.

11. Prozess (100, 200, 300) nach Anspruch 10, bei dem der Teilstrom (12) oder ein in einer Spurenentfernung (90) hieraus gebildeter Folgestrom zumindest zu einem Teil als das zweite Komponentengemisch der ersten oder der zweiten Fraktionierung (10a, 10b, 90a) unterworfen wird.

12. Prozess (100, 200, 300) nach einem der vorstehenden Ansprüche, bei dem in das Synthesegaserzeugungsverfahren (20) Kohlendioxid eingespeist wird.

13. Prozess (100, 200, 300) nach einem der vorstehenden Ansprüche, bei dem in dem Dampfspaltverfahren (10) und/oder in dem Synthesegaserzeugsverfahren (20) eine elektrische Beheizung durchgeführt wird, und bei dem in das Synthesegaserzeugungsverfahren (20) Methan eingespeist wird, das in dem Dampfspaltverfahren (10) gebildet wird.

14. Prozess (100, 200, 300) nach einem der vorstehenden Ansprüche, bei dem eine oder mehrere Pumpen, ein oder mehrere Kompressoren und/oder eine oder mehrere Turbinen verwendet werden, die zumindest teilweise elektrisch angetrieben werden.

15. Prozess (100, 200, 300) nach einem der vorstehenden Ansprüche, bei dem der erste Stoffstrom (10) unter Verwendung einer Tailendhydrierung gebildet wird und Wasserstoff enthält.

16. Prozess (100, 200, 300) nach einem der vorstehenden Ansprüche, bei dem der Olefineffizienzfaktor und/oder Monomerwirkungsgrad mindestens 100%, insbesondere mindestens 115%, 125% oder 130%, und bis zu 150%, beträgt.

**Claims**

1. Process (100, 200, 300) for preparing propylene, wherein a first material stream (1) is provided using a steam cracking method (10) and one or more fractionations (10a, 10b, 90a), which first material stream is rich in ethylene, wherein a second material stream (2) is provided using a synthesis gas production method (20), which second material stream contains carbon monoxide and hydrogen, wherein at least part of the ethylene from the first material stream (1) is reacted with at least part of the carbon monoxide and the hydrogen from the second material stream (2) to form propanal using a hydroformylation (30) to obtain a third material stream (3), and wherein at least part of the propanal in the third material stream (3) is converted to propylene, the ethylene being provided by means of the steam cracking method (10) in a first component mixture, the propylene being provided in a second component mixture, **characterized in that** at least part of the first component mixture is subjected to a first fractionation (10a, 10b) to obtain the first material stream, and **in that** at least part of the second component mixture is subjected to one or more fractionation steps of the first fractionation (10a, 10b) or a second fractionation (90a) to obtain a pure propylene stream (19).

2. Process (100, 200, 300) according to claim 1, wherein the reaction of at least part of the propanal to form the propylene comprises a hydrogenation (50) to give propanol and a dehydrogenation (60) to give propylene to obtain the second product mixture.

3. Process (100, 200, 300) according to claim 2, wherein the third material stream (3) contains unreacted ethylene, hydrogen and carbon monoxide as light components, the light components being transferred in a separation (40) at least in part into a fourth material stream (4).

4. Process (100, 200, 300) according to claim 3, wherein the separation (40) of at least part of the light components is carried out before the hydrogenation (50) or between the hydrogenation (50) and the dehydrogenation (60).

5. Process (100, 200, 300) according to either claim 3 or claim 4, wherein at least part of the fourth material stream (4) is returned into the process (100, 200, 300) as a recycle stream (5) upstream of the hydroformylation (30).

6. Process (100, 200, 300) according to claim 4, wherein the return of at least part of the fourth material stream (4) comprises a post-compression.

7. Process (100, 200, 300) according to claim 6, wherein part of the fourth material stream (4) is returned to the synthesis gas production method (20).

8. Process (100, 200, 300) according to either claim 5 or claim 6, wherein the separation (40) of at least part of the light components comprises an adsorptive separation, an adsorptive separation, a membrane separation, a separation by distillation and/or a phase separation.

9. Process (100, 200, 300) according to any of claims 2 to 8, wherein a hydrogen stream (8) containing hydrogen is fed into the hydrogenation and is provided using a separate hydrogen source and/or is obtained using the synthesis gas production method (20) and/or using the steam cracking method (10) and is separated using a hydrogen removal (70).

10. Process (100, 200, 300) according to any of claims 2 to 9, wherein a product stream (10) containing water, propylene and secondary components is formed in the dehydrogenation (60) and is subjected to a water removal (80) to obtain the partial stream (12) which contains propylene and also the secondary components and which is depleted of water.

11. Process (100, 200, 300) according to claim 10, wherein the partial stream (12) or a subsequent stream formed therefrom in a trace removal (90) is subjected at least partly as the second component mixture to the first or second fractionation (10a, 10b, 90a).

12. Process (100, 200, 300) according to any of the preceding claims, wherein carbon dioxide is fed into the synthesis gas production method (20).

13. Process (100, 200, 300) according to any of the preceding claims, wherein electrical heating is performed in the steam cracking method (10) and/or in the synthesis gas production method (20), and wherein methane formed in the steam cracking method (10) is fed into the synthesis gas production method (20).

14. Process (100, 200, 300) according to any of the preceding claims, wherein one or more pumps, one or more compressors and/or one or more turbines are used that are at least partially electrically driven.

15. Process (100, 200, 300) according to any of the preceding claims, wherein the first material stream (10) is formed using a tail-end hydrogenation and contains hydrogen.

16. Process (100, 200, 300) according to any of the preceding claims, wherein the olefin efficiency factor and/or monomer efficiency is at least 100%, in particular at least 115%, 125% or 130%, and up to 150%.

**Revendications**

1. Processus (100, 200, 300) pour la fabrication de propylène, dans lequel en utilisant un procédé de craquage à la vapeur (10) et un ou plusieurs fractionnements (1 0a, 10b, 90a) un premier flux de matières (1) est fourni, qui est riche en éthylène, dans lequel en utilisant un procédé de production de gaz de synthèse (20), un deuxième flux de matières (2) est fourni, qui contient du monoxyde de carbone et de l'hydrogène, dans lequel au moins une partie de l'éthylène provenant du premier flux de matières (1) avec au moins une partie du monoxyde de carbone et de l'hydrogène provenant du deuxième flux de matières (2) est converti en propanal en utilisant une hydroformylation (30) pour obtenir un troisième flux de matières (3), et dans lequel au moins une partie du propanal dans le troisième flux de matières (3) est converti en propylène, dans lequel l'éthylène est fourni au moyen du procédé de craquage à la vapeur (10) dans un premier mélange de composants, dans lequel le propylène est fourni dans un second mélange de composants, **caractérisé en ce qu'**au moins une partie du premier mélange de composants est soumis à un premier fractionnement (10a, 10b) pour obtenir le premier flux de matières, et **en ce qu'**au moins une partie du second mélange de composants est soumis à une ou plusieurs étapes de fractionnement du premier fractionnement (10a, 10b) ou à un second fractionnement (90a) pour obtenir un flux de propylène pur (19).

2. Processus (100, 200, 300) selon la revendication 1, dans lequel la conversion d'au moins une partie du propanal en le propylène comprend une hydrogénation (50) en propanol et une déshydratation (60) en propylène pour obtenir le second mélange de produits.

3. Processus (100, 200, 300) selon la revendication 2, dans lequel le troisième flux de matières (3) contient de l'éthylène non converti, de l'hydrogène et du monoxyde de carbone en tant que composants légers, dans lequel les composants légers sont transférés dans une séparation (40) au moins en partie dans un quatrième flux de matières (4).

4. Processus (100, 200, 300) selon la revendication 3, dans lequel la séparation (40) d'au moins une partie des composants légers est effectuée avant l'hydrogénation (50) ou entre l'hydrogénation (50) et la déshydratation (60).

5. Processus (100, 200, 300) selon la revendication 3 ou 4, dans lequel au moins une partie du quatrième flux de matières (4) est renvoyé sous forme de flux de recyclage (5) en amont de l'hydroformylation (30) dans le processus (100, 200, 300).

6. Processus (100, 200, 300) selon la revendication 4, dans lequel le renvoi d'au moins une partie du quatrième flux de matières (4) comprend une recompression.

7. Processus (100, 200, 300) selon la revendication 6, dans lequel une partie du quatrième flux de matières (4) est renvoyé au procédé de production de gaz de synthèse (20).

8. Processus (100, 200, 300) selon l'une des revendications 5 ou 6, dans lequel la séparation (40) d'au moins une partie des composants légers comprend une séparation par adsorption, une séparation adsorbante, une séparation par membrane, une séparation par distillation et/ou une séparation de phases.

9. Processus (100, 200, 300) selon l'une des revendications 2 à 8, dans lequel dans l'hydrogénation, un flux d'hydrogène (8) est alimenté, qui contient de l'hydrogène, qui est fourni en utilisant une source d'hydrogène séparée et/ou obtenu en utilisant le procédé de production de gaz de synthèse (20) et/ou en utilisant le procédé de craquage à la vapeur (10) et séparé en utilisant une séparation d'hydrogène (70).

10. Processus (100, 200, 300) selon l'une des revendications 2 à 9, dans lequel dans la déshydratation (60), un flux de produits (10) contenant de l'eau, du propylène et des composants secondaires est formé, qui est soumis pour obtenir le propylène et en outre le flux partiel (12) contenant les composants secondaires et appauvri en eau à une séparation d'eau (80).

11. Processus (100, 200, 300) selon la revendication 10, dans lequel le flux partiel (12) ou un flux de suite formé à partir de celui-ci dans une suppression des traces (90) est soumis au moins en partie sous forme du second mélange de composants au premier ou au second fractionnement (10a, 10b, 90a).

12. Processus (100, 200, 300) selon l'une quelconque des revendications précédentes, dans lequel dans le procédé de production de gaz de synthèse (20), du dioxyde de carbone est injecté.

**13.** Processus (100, 200, 300) selon l'une quelconque des revendications précédentes, dans lequel dans le procédé de craquage à la vapeur (10) et/ou dans le procédé de production de gaz de synthèse (20), un chauffage électrique est réalisé et dans lequel dans le procédé de production de gaz de synthèse (20), du méthane est injecté, qui est formé dans le procédé de craquage à la vapeur (10).

**14.** Processus (100, 200, 300) selon l'une quelconque des revendications précédentes, dans lequel une ou plusieurs pompes, un ou plusieurs compresseurs et/ou une ou plusieurs turbines sont utilisé(e)s, qui sont entraîné(e)s au moins partiellement par voie électrique.

**15.** Processus (100, 200, 300) selon l'une quelconque des revendications précédentes, dans lequel le premier flux de matières (10) est formé en utilisant une hydratation de la queue et contient de l'hydrogène.

**16.** Processus (100, 200, 300) selon l'une quelconque des revendications précédentes, dans lequel le facteur d'efficacité des oléfines et/ou le rendement des monomères est d'au moins 100 %, en particulier d'au moins 115 %, 125 % ou 130 %, et jusqu'à 150 %.

Fig. 1

**Fig. 2**

EP 3 922 621 B1

**Fig. 3**

EP 3 922 621 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 10519087 B2

- US 9856198 B

**In der Beschreibung aufgeführte Nicht-Patentliteratur**

- Propylene" in Ullmann's Encyclopedia of Industrial Chemistry. 2012
- Ethylene" in Ullmann's Encyclopedia of Industrial Chemistry. 15. April 2009

- Gas Production" in Ullmann's Encyclopedia of Industrial Chemistry, beispielsweise die Publikation vom. 15. Dezember 2006